(19) Europäisches Patentamt
European Patent Office
Office européen des brevets

(11) Veröffentlichungsnummer: **0 032 674**
**A1**

(12) # EUROPÄISCHE PATENTANMELDUNG

(21) Anmeldenummer: **81100068.6**

(22) Anmeldetag: **08.01.81**

(51) Int. Cl.³: **C 07 C 157/14**, A 01 N 47/42, C 23 F 11/16

(30) Priorität: **19.01.80 DE 3001853**

(43) Veröffentlichungstag der Anmeldung: **29.07.81 Patentblatt 81/30**

(84) Benannte Vertragsstaaten: **BE CH DE FR GB IT LI NL SE**

(71) Anmelder: **HOECHST AKTIENGESELLSCHAFT, Postfach 80 03 20, D-6230 Frankfurt/Main 80 (DE)**

(72) Erfinder: **Diery, Helmut, Dr., Theresenstrasse 45, D-6233 Kelkheim (Taunus) (DE)**
Erfinder: **Wagemann, Wolfgang, Dr., Beektwiete 2a, D-2071 Tremsbüttel (DE)**
Erfinder: **Bücking, Hans-Walter, Dr., In den Padenwiesen 30, D-6233 Kelkheim (Taunus) (DE)**
Erfinder: **Hille, Martin, Dr., In den Eichen 46, D-6237 Liederbach (DE)**
Erfinder: **Wallhäusser, Karl Heinz, Prof. Dr., Lessingstrasse 20, D-6238 Hofheim am Taunus (DE)**

(54) **Alkyletherisothiuroniumsalze, Verfahren zu deren Herstellung und deren Verwendung als Korrosionsinhibitoren und Mikrobiozide.**

(57) Alkyletherisothiuroniumsalze der allgemeinen Formel I

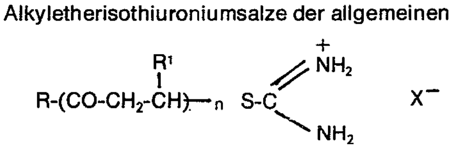

I

worin R $C_8$-$C_{30}$-Alkyl, $C_8$-$C_{30}$-Alkenyl, $C_8$-$C_{30}$-Hydroxialkyl oder Phenyl oder Naphthyl, die durch 1 bis 3 $C_4$-$C_{12}$-Alkylgruppen substituiert sind, $R_1$ Wasserstoff, Methyl oder Ethyl, n Zahlen von 1 bis 20 und X ein Anion bedeutet, Verfahren zu deren Herstellung und deren Verwendung als Korrosionsinhibitoren und Mikrobiozide.

EP 0 032 674 A1

ACTORUM AG

HOECHST AKTIENGESELLSCHAFT HOE 80/F 009 Dr.OT/jk

# Alkyletherisothiuroniumsalze, Verfahren zu deren Herstellung und deren Verwendung als Korrosionsinhibitoren und Mikrobiozide

Bei der Rohölproduktion wird während der Anfangsphase der Förderung praktisch reines Rohöl gefördert und es treten keine Schäden durch Korrosion oder Bakterienbefall auf. Jedes Ölfeld fördert aber von einem bestimmten Zeitpunkt an zunehmende Mengen Wasser, das emulsionsartig im Rohöl verteilt ist. Für den Transport und die Verarbeitung muß diese Wasser-in-Öl-Emulsion gespalten und das Wasser abgetrennt werden. Das separierte Wasser wird über ein Verteilungssystem wieder in die Lagerstätte eingepreßt. Von dieser Phase ab treten je nach der Korrosivität des Lagerstättenwassers, was vornehmlich von dessen Elektrolyt-, Kohlendioxid- und Schwefelwasserstoffgehalt abhängt, Korrosionsschäden in den Feldleitungen, Aufbereitungsanlagen und dem Flutwassersystem auf. In diesen Anlagenteilen siedeln sich häufig Bakterien an, die die Korrosion verstärken und Stoffwechselprodukte bilden, die das Einpressen des Wassers in die meist fein porösen Lagerstätten erschweren. Die größten Schäden verursachen sulfatreduzierende Bakterien, wie Desulfovibrio desulfuricans, welche wasserlösliche Sulfate zu Schwefelwasserstoff reduzieren. Dieser erhöht die Korrosivität der Wässer. Neben den aus den Rohölen abgetrennten Wässern werden große Mengen Wasser auch aus Wasserhorizonten, Oberflächenwässer und Meerwasser zur Erhöhung der Förderleistung in die Öllagerstätten eingepreßt. Dabei müssen besonders Oberflächenwässer und Meerwasser aufbereitet werden, um die Korrosion und den Bakterienbefall minimal zu halten. In vielen Fällen ist es unumgänglich, diese Wässer nach der Aufbereitung bzw. Separierung von Rohöl mit keimtötenden Mitteln zu behandeln.

Zu diesem Zweck sind neben Chlorphenolen und niederen Aldehyden besonders kationische Tenside wie z. B. Alkylpropylendiamine, quartäre Ammoniumverbindungen und Alkylguanidine im Einsatz. Diese kationischen Tenside haben den großen Vorteil, daß sie gleichzeitig die Korrosion inhibieren. Dadurch werden die Leitungssysteme und Anlagen geschützt und deren Funktionsdauer verlängert. Es fallen außerdem signifikant geringere Mengen von Korrosionsprodukten, Bakterienrückständen sowie deren Stoffwechselprodukten an, welche sonst zu Verstopfungen der Einpreßhorizonte führen würden.

Aufgrund der dispergierenden Wirkung der kationischen Tenside werden kleine Schmutzteilchen, z.B. Folgeprodukte der Korrosion und des mikrobiologischen Wachstums in Schwebe gehalten und das Leitungssystem bleibt sauber. Es müssen dabei stets ausreichend lösliche kationische Tenside verwendet werden, weil es durch Adsorption an Feststoffteilchen zur Bildung von Schlamm kommen kann. Mikroorganismen neigen auch dazu, sich an bestimmte Konzentrationen von Bakteriziden zu gewöhnen. Durch gelegentlichen Wechsel zu einer möglichst stark anders strukturierten Wirksubstanz kann man diese Resistenzbildung verhindern.

Es wurde nun gefunden, daß Alkyletherisothiuroniumsalze eine verbesserte Wirkung als Korrosionsinhibitoren und gleichzeitig als Mikrobiozid zeigen. Gegenstand der Erfindung sind somit Alkyletherisothiuroniumsalze der allgemeinen Formel I

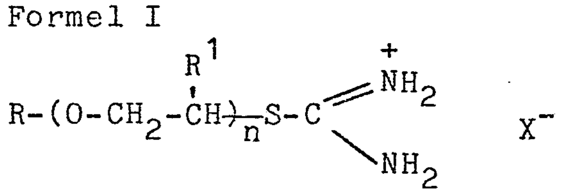 I

worin R $C_8$-$C_{30}$-Alkyl, $C_8$-$C_{30}$-Alkenyl, $C_8$-$C_{30}$-Hydroxialkyl oder Phenyl oder Naphthyl, die durch 1 bis 3 $C_4$-$C_{12}$-Alkylgruppen substituiert sind, $R^1$ Wasserstoff, Methyl oder Ethyl, n Zahlen von 1 bis 10 und X ein Anion bedeuten.

Die erfindungsgemäßen Verbindungen werden hergestellt, indem man oxalkylierte Alkohole bzw. Phenole der Formel II

$$R\!-\!(O\!-\!CH_2\!-\!\underset{}{\overset{R^1}{\overset{|}{C}}}H)_n\!-\!OH \qquad II$$

zunächst mit einem Halogenierungsagens, vorzugsweise Thionylchlorid, umsetzt.

Die Reaktion kann in Lösungsmitteln wie aliphatischen oder aromatischen Kohlenwasserstoffen oder aliphatischen oder aromatischen Ethern durchgeführt werden. Vorzugsweise arbeitet man jedoch ohne Lösungsmittel. Die vorteilhafte Reaktionstemperatur liegt bei 40 bis 80°C.

Die so erhaltenen Halogenide isoliert man durch Abdestillieren der Lösungsmittel bzw. sie fallen beim Arbeiten in Substanz direkt an. Die bei der Halogenierungsreaktion sich bildenden sauren Gase werden mit Stickstoff aus dem Reaktionsgemisch vertrieben. Zur Erzielung einer ansprechenden Farbe der Produkte kann man bei der Umsetzung etwas Aktivkohle zusetzen, die gleichzeitig noch katalysierend wirkt.

Die Ausgangsverbindungen der Formel II werden durch Umsetzung von Alkoholen mit Alkylenoxiden, vorzugsweise Ethylenoxid, nach bekannten Verfahren synthetisiert. Selbstverständlich können auch Gemische von Alkylenoxiden sowie verschiedene Alkylenoxide nacheinander verwendet werden. Als Alkohole werden vorzugsweise Fettalkohole oder Alkylphenole eingesetzt, die man in Form der reinen Verbindungen, vorzugsweise jedoch in Form ihrer großtechnisch anfallenden Kettenschnitte verwendet. Solche technischen Produkte sind z. B. Lauryl-, Cocosfett- oder Talgfettalkohol bzw. entsprechende synthetische Ziegler- oder Oxoalkohle. Auf der Basis von Alkylphenolen sind Octyl-, Nonyl- und Triisobutylphenol die bevorzugt verwendeten Komponenten.

Die in erster Stufe synthetisierten Etherhalogenide werden zur Herstellung der erfindungsgemäßen Verbindungen der Formel I mit Thioharnstoff umgesetzt. Zweckmäßigerweise arbeitet man in für die Reaktion indifferenten Lösungsmitteln, vorzugsweise in niederen Alkoholen oder anderen polaren Lösungsmitteln wie z.B. Isopropanol, Isobutanol oder Dimethylformamid. Die bevorzugte Reaktionstemperatur liegt bei 70 - 110°C. Bei dieser Herstellung erhält man immer Verbindungen der Formel I, wo X ein Halogenid-ion, vorzugsweise Chlorid bedeutet. Aus diesem Halogenid lassen sich durch Austausch andere Salze gewinnen, beispielsweise das Sulfat und andere anorganische Salze. Es ist aber ebenso möglich, das Halogenid-ion gegen Ionen organischer Säuren auszutauschen, so daß man beispielsweise das Acetat, Lactat oder Oxalat erhält. Durch Abdestillieren der Lösungsmittel können die reinen Verbindungen der Formel I isoliert werden. Sie sind grenzflächenaktiv und in Wasser und niederen Alkoholen löslich. In Alkoholen lassen sich noch in hoher Konzentration klare flüssige Zubereitungen herstellen.

Es wurde nun überraschend gefunden, daß die erfindungsgemäßen Verbindungen eine ausgezeichnete mikrobiozide und unter anaeroben Bedingungen auch eine korrosionsinhibierende Wirkung zeigen. Aufgrund ihrer guten Löslichkeit und Salzverträglichkeit kann bei der Herstellung von Desinfektionsmittel-Formulierungen auf zusätzliche Emulgatoren verzichtet werden, welche die mikrobiozide Wirkung, wie allgemein bekannt ist, beeinträchtigen können.

Flüssige Zubereitungen solcher mikrobioziden Mittel werden im allgemeinen in Wasser oder niederen Alkoholen wie z.B. Isopropanol oder Wasser/Alkohol-Gemischen hergestellt. Sie enthalten 5 bis 70 Gew.-%, vorzugsweise 20 bis 55 Gew.-% der erfindungsgemäßen Alkyletherthiuroniumsalze. Für die Herstellung solcher flüssigen Zubereitungen kann man direkt von den Lösungen ausgehen, die bei der Synthese dieser Alkyletherisothiuroniumsalze anfallen. Man verdünnt diese

Lösungen durch Zugabe von Wasser bzw. Alkohol auf die gewünschte Endkonzentration. Selbstverständlich können in solchen Formulierungen außerdem noch weitere Substanzen und Hilfsmittel enthalten sein, wie sie üblicherweise in solchen Zubereitungen mitverwendet werden. Hierzu gehören z.B. kationische oder nichtionische oberflächenaktive Substanzen, Elektrolyte, Komplexbildner, Lösungsvermittler, niedere Aldehyde wie z.B. Formaldehyd, Glyoxal oder Glutardialdehyd sowie Farb- und Duftstoffe. Diese Zusätze dienen beispielsweise zur Verbesserung des Netzvermögens, der Härtestabilität, zur Viskositätseinstellung und zur Erhöhung der Kältestabilität der Lösungen.

Beispiel 1:

590 Gewichtsteile eines mit 6 Mol Ethylenoxid umgesetzten Oxoalkohols (Kettenbereich $C_{12}$-$C_{15}$, linearer Anteil ca. 60 %) und 11 Gewichtsteile Aktivkohle werden auf ca. 80°C erwärmt.

Über 35 Minuten werden 446 Gewichtsteile Thionylchlorid bei dieser Temperatur zugetropft. Anschließend wird unter Durchleiten von Stickstoff 2 Stunden bei 110°C nachgerührt, wobei überschüssiges Thionylchlorid abdestilliert. Nach dem Abkühlen auf 50°C werden 20 Gewichtsteile Waser zugesetzt und mit wasserfreier Soda bis zur neutralen Reaktion versetzt und abgesaugt.

Man erhält 610 Gewichtsteile eines klaren, gelben, flüssigen Produktes. Es wird in 700 Teilen Isobutanol gelöst, mit 95 Teilen Thioharnstoff versetzt und zwei Stunden am Rückfluß gekocht. Es resultiert eine klare orangegelbe 50 %ige Lösung des Isothiuroniumsalzes der Formel

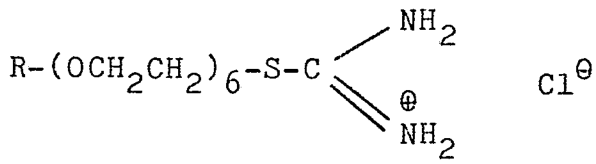

R = $C_{12}$-$C_{15}$-Alkyl

Beispiel 2:

364 Gewichtsteile eines mit 4 Mol Ethylenoxid umgesetzten Nonylphenols und 5,5 Gewichtsteile Aktivkohle werden auf 80°C erwärmt. Über 55 Minuten werden 167 Gewichtsteile Thionylchlorid zugetropft. Es findet sofort eine starke Entwicklung von $SO_2$ und HCl statt. Die Gase werden über Waschflaschen in NaOH absorbiert.

Nach dem Zutropfen wird 2 Stunden bei 100°C unter Durchleiten eines mittelstarken $N_2$-Stromes nachgerührt. Man läßt auf 50°C abkühlen, fügt 10 Gew.-T. Wasser hinzu und neutralisiert portionsweise mit 20 Gew.-T. wasserfreier Soda. Nach dem Absaugen erhält man 380 Gewichtsteile eines klaren hellgelben, flüssigen Produktes. Man nimmt in 450 Gew.-T. Isobutanol auf, fügt 70 Gew.-T. Thioharnstoff zu und erhitzt 4 Stunden am Rückfluß.

Es resultiert eine klare orangegelbe 50 %ige Lösung des Isothiuroniumsalzes der Formel

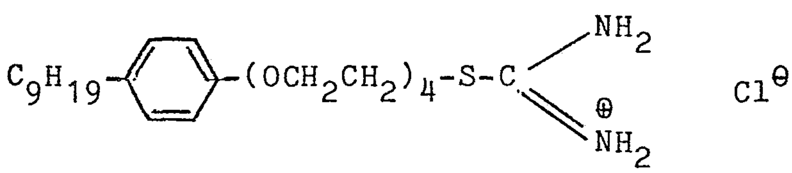

Beispiel 3:

Analog Beispiel 2 werden umgesetzt:
681 Gewichtsteile eines mit 3 Mol Ethylenoxid umgesetzten Oxoalkohols (Kettenbereich $C_{12}$-$C_{15}$, linearer Anteil ca. 60 %), 13 Gewichtsteile Aktivkohole, 333 Gewichtsteile Thionylchlorid. Man erhält nach Versetzen mit 20 Gew.-T. Wasser und Neutralisation mit 40 Gew.-T. Soda 700 g flüssiges Chlorid

das in 850 Gew.-T. Isobutanol aufgenommen und mit 150 Gew.-T. Thioharnstoff analog Beispiel 2 umgesetzt wird. Man erhält so die Verbindung der Formel

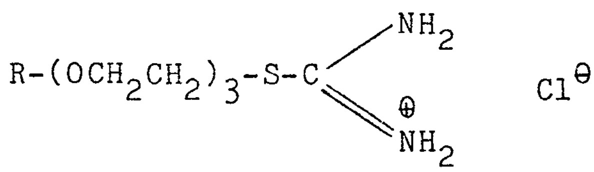

R = $C_{12}$-$C_{15}$-Alkyl

Beispiel 4:

276 Gewichtsteile eines mit 2 Mol Ethylenoxid umgesetzten synthetischen linearen Fettalkohols (Kettenverteilung 55 % $C_{12}$, 45 % $C_{14}$) und 7 Gewichtsteilen Aktivkohle werden auf 50°C erwärmt. 342 Gewichtsteile Thionylchlorid werden über 45 Minuten zugetropft. Die Reaktion ist exotherm, entweichendes $SO_2$ und HCl wird über Waschflaschen in NaOH absorbiert.

Es wird 2 Stunden bei 80°C nachgerührt, anschließend überschüssiges Thionylchlorid im Vakuum abdestilliert. Es wird abgesaugt, mit 300 Vol. Teilen gesättigter Sodalösung versetzt und im Scheidetrichter getrennt. Man erhält 270 Gewichtsteile gelbes flüssiges Chlorid. Es wird in 330 Gewichtsteilen Isobutanol aufgenommen, mit 65 Gewichtsteilen Thioharnstoff versetzt und 4 Stunden am Rückfluß erhitzt. Es resultiert eine klare 50 %ige Lösung des Isothiuroniumsalzes der Formel

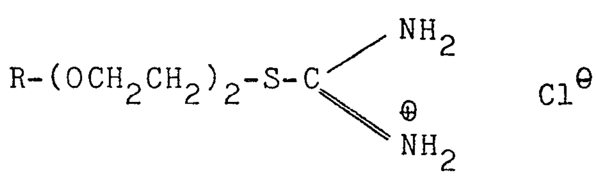

R = $C_{12}$-$C_{14}$-Alkyl

Beispiel 5:

330 Gewichtsteile eines mit 2,5 Mol Ethylenoxid umgesetzten Nonylphenols und 7 Gew.-T. Aktivkohle werden auf 50°C erwärmt. Innerhalb von 1 Stunde tropft man 288 Gew.-T. Thionylchlorid zu.
Die Reaktion ist exotherm bei heftiger Gasentwicklung. $SO_2$ und HCl werden in NaOH absorbiert. Nach dem Zutropfen wird 2 Stunden bei Rückflußtemperatur nachgerührt. Überschüssiges Thionylchlorid wird im Vakuum abdestilliert. Danach wird bei 100°C 2 Stunden $N_2$ durch die Reaktionsmischung geleitet und abgesaugt. Anschließend wird Vakuum angelegt und bei 80°C nochmals 2 Stunden Stickstoff zur Vertreibung von sauren Restbestandteilen durch die Lösung geperlt.

Man erhält 330 Gewichtsteile eines schwach gelben klarflüssigen Produktes, das in 400 Gewichtsteilen Isobutanol aufgenommen wird. Nach Zugabe von 70 Gewichtsteilen Thioharnstoff wird 4 Stunden am Rückfluß gekocht. Es resultiert eine klare, orangefarbene 50 %ige Lösung des Isothiuroniumsalzes der Formel

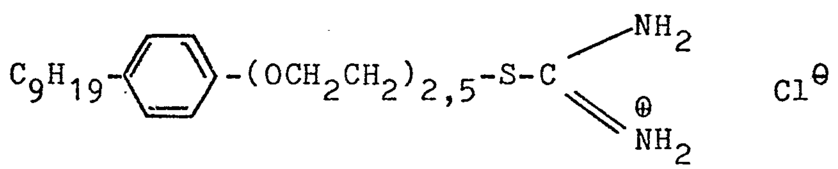

Beispiel 6 (Korrosioninhibierung):

Zur Prüfung der Inhibierwirkung wurden Stahl-Coupons (1 x 10 cm) bei 80°C 7 Stunden in gerührtes, ständig von Kohlendioxid durchperltes salines Wasser (200 g/l) getaucht und anschließend der Gewichtsverlust bestimmt. Das Wasser enthält unter diesen Bedingungen unter 0,5 ppm Sauerstoff. Der Schutzwert wird in % angegeben und ist die Minderung des Metallabtrages gegenüber dem Blindwert.

Tabelle I

| Produkt aus Beispiel | % Schutz bei Einsatzmengen von 5 ppm | von 25 ppm |
|---|---|---|
| 2 | 86 | 94 |
| 3 | 85 | 93 |
| 4 | 85 | 92 |
| 5 | 87 | 94 |
| Vergleichsprodukt | 84 | 90 |

Vergleichsprodukt: Cocosalkyl-dimethyl-benzyl-ammonium-chlorid

Beispiel 7:

(bakterizide Wirkung gegen Desulfovibrio desulfuricans - Suspensionsversuch mit ca. $10^6$ Keimen/ml, Einwirkungszeit 24 Stdn. bei 20°C, Angabe der minimalen mikrobiziden Konzentration in $\mu$g/ml):

| | | |
|---|---|---|
| Produkt aus Beispiel | 1 | 16 |
| " " " | 2 | 16 |
| " " " | 3 | 16 |
| " " " | 4 | 14 |
| " " " | 5 | 16 |

Zum Vergleich:

| | |
|---|---|
| Cocosalkyl-dimethyl-benzylammonium-chlorid | 64 |
| Didecyl-dimethyl-ammoniumchlorid | 64 |
| Bis-(p-chlorphenyl-diguanidin)-1,6-hexan-di-chlorid | 25 |
| p-Chlor-metakresol | 50 |
| 1-(3-Chlorallyl)3,5,7-triaza-1-azonia-adamantanchlorid | 100 |
| Cocosalkylpropylendiamin-acetat | 25 |

## Beispiel 8:

Mikrobizide Wirkung von Alkyletherisothiuroniumsalzen im Vergleich zu anderen antimikrobiellen Wirkstoffen - Suspensionsversuch mit ca. $10^6$ Keimen/ml, Einwirkungszeit 24 Stunden bei 20°C, Angabe der minimalen mikrobioziden Konzentration in µg/ml.

| Verbindung | Staph. aur. | E.coli | Ps. aerug. | Prot. vulg. | C.alb. | Grünalgen |
|---|---|---|---|---|---|---|
| A | 125 | 4 | 32 | 32 | 62 | 8 |
| B | 32 | 8 | 32 | 32 | 16 | 8 |
| C | 125 | 8 | 1000 | 125 | 16 | 8 |
| D | 125 | 16 | 250 | 250 | 16 | 8 |
| zum Vergleich: | | | | | | |
| 1 | 32 | 64 | 125 | 125 | 64 | 8 |
| 2 | 4 | 32 | 64 | 64 | 32 | 8 |
| 3 | 100 | 100 | 100 | 100 | 12 | 16 |
| 4 | 100 | 400 | 200 | 100 | 200 | - |
| 5 | 100 | 200 | 400 | 200 | 400 | - |
| 6 | 25 | 50 | 50 | 50 | 50 | 50 |

Die Verbindungen haben folgende Struktur:

erfindungsgemäße Verbindungen nach Formel I

A: R = p-Nonylphenyl
R' = H
n = 2,5
X = Chlorid

B: R = p-Nonylphenyl
R' = H
n = 4
X = Chlorid

C: R = $C_{12}$-$C_{15}$-Alkyl, ca. 60 % linear
R' = H
n = 3
X = Chlorid

D: R = Laurylalkyl linear, 55 % $C_{12}$, 45 % $C_{14}$
R' = H
n = 2
X = Chlorid

Vergleichsprodukte:

1 Alkyldimethylbenzylammoniumchlorid
2 Didecyl-dimethyl-ammoniumchlorid
3 Bis(p-chlorphenyl-diguanidin)-1,6-hexan-di-chlorid
4 p-Chlor-m-kresol
5 1-(3-Chlorallyl)-3,5,7-tri-aza-1-azonia-adamantan-chlorid
6 Alkylpropylendiamin-acetat

Beispiel 9

Bakterizide Wirkung in einer Desinfektionsreiniger-Formulierung zur Flächendesinfektion - Beimpfung von 50 x 50 mm großen Stücken aus Schichtpreßstoff, PVC-Boden oder Fliesen, Keimgehalt pro ml CSL-Suspension: $10^8$ - $10^9$. Nach Antrocknung der Keimsuspension (90 Min.) wurden die Flächen aus 30 - 40 cm Entfernung mit dem Mittel besprüht.

Formulierungen:

| | | |
|---|---|---|
| 1. | 9,7 % | Verbindung A gemäß Beispiel 8, als 50 %ige Lösung in einem 2:1-Gemisch Isobutanol/Isopropanol |
| | 5 % | Nonylphenol - 10 EO-polyglykolether |
| | ad 100 % | Wasser |

2. 9,7 % Verbindung A wie vorstehend
5 % Nonylphenol- 10 EO-polyglykolether
5 % Formalin 35 %ig
4 % Glyoxal 40 %ig
5 % Isopropanol
ad 100 % Wasser

| Formulierung (1:10 $H_2O$-verdünnt) | Keimreduzierung* (KBE) Staph. aur. 1 | 2 | 4 | 6 Stdn. | Klebs. pneum. 1 | 2 | 4 | 6 Stdn. |
|---|---|---|---|---|---|---|---|---|
| 1 | 0 | 0 | 0 | 0 | 70 | 10 | 50 | 50 |
| 2 | 0 | 0 | 0 | 0 | 0 | 0 | 0 | 0 |

* entsprechend den Empfehlungen für die Prüfung und Bewertung der Wirksamkeit chemischer Desinfektionsverfahren (Zbl.Bakt.Hyg. I, Abt. Orig. B 105, 335 - 380 (1977).

Patentansprüche:

1. Alkyletherisothiuroniumsalze der allgemeinen Formel I

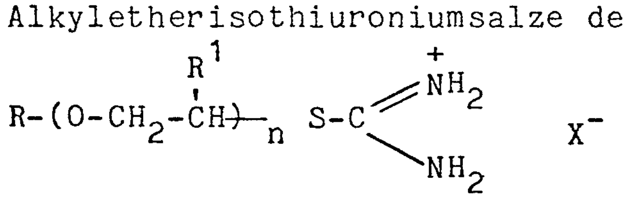 I

worin R $C_8$-$C_{30}$-Alkyl, $C_8$-$C_{30}$-Alkenyl, $C_8$-$C_{30}$-Hydroxialkyl oder Phenyl oder Naphthyl, die durch 1 bis 3 $C_4$-$C_{12}$-Alkylgruppen substituiert sind, $R_1$ Wasserstoff, Methyl oder Ethyl, n Zahlen von 1 bis 20 und X ein Anion bedeutet.

2. Verfahren zur Herstellung der Alkyletherisothioroniumsalze nach Anspruch 1, dadurch gekennzeichnet, daß man eine Verbindung der Formel

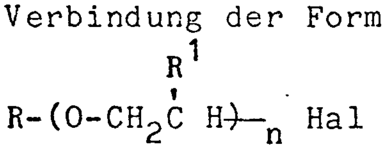

worin Hal ein Halogenatom bedeutet und R, $R^1$ und n die oben genannten Bedeutungen haben, mit Thioharnstoff umsetzt.

3. Verwendung der Alkyletherisothiuroniumsalze nach Anspruch 1 als mikrobiozide Mittel.

4. Verwendung der Alkyletherisothiuroniumsalze nach Anspruch 1 als korrosionsinhibierende Mittel.

Europäisches Patentamt

# EUROPÄISCHER RECHERCHENBERICHT

0032674

Nummer der Anmeldung
EP 81 10 0068

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 2 547 366 (LOUIS H. BOCK) * Insgesamt * -- | 1-3 |
| A | US - A - 2 624 762 (PAUL M. DOWNEY) * Insgesamt * -- | 1,4 |
| A | CH - A - 374 982 (CHEMISCHE WERKE ALBERT) * Ansprüche; Seite 1 * ---- | 1-3 |

KLASSIFIKATION DER ANMELDUNG (Int Cl.)
C 07 C 157/14
A 01 N 47/42
C 23 F 11/16

RECHERCHIERTE SACHGEBIETE (Int. Cl.)
C 07 C 157/14
C 23 F 11/16

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-04-1981 | HENRY |

EPA form 1503.1 06.78